# EUROPEAN PATENT SPECIFICATION

(11) **EP 1 445 269 B1**
(45) Date of publication and mention of the grant of the patent: **18.03.2009**
(21) Application number: 04001733.7
(22) Date of filing: 27.01.2004
(51) Int. Cl.: C08G 18/02

(54) **Polycarbodiimide having high index of refraction and production method thereof**
Polycarbodiimide mit hohem Brechungsindex und Verfahren zu ihrer Hestellung
Polycarbodiimide à indice de réfraction elevé et son procédé de fabrication

(30) Priority: 10.02.2003 JP 2003032929
(43) Date of publication of application: 11.08.2004
(73) Proprietor: NITTO DENKO CORPORATION, Osaka (JP)
(72) Inventor: Sadayori, Naoki, Ibaraki-shi Osaka (JP); Hotta, Yuji, Ibaraki-shi Osaka (JP)
(74) Representative: Grünecker, Kinkeldey, Stockmair & Schwanhäusser Anwaltssozietät

(56) References cited:
- EP-A- 1 125 956
- EP-A- 1 385 027
- EP-A- 1 439 406
- GB-A- 851 936
- PATENT ABSTRACTS OF JAPAN vol. 1996, no. 12, 26 December 1996 (1996-12-26) & JP 08 208788 A (MITSUI TOATSU CHEM INC), 13 August 1996 (1996-08-13)

## Description

This invention relates to a polycarbodiimide copolymer which has excellent heat resistance and chemical resistance and also has high index of refraction, and a production method thereof.

In recent years, studies on transparent polymers having high index of refraction have been carried out extensively, and they have been used for thin spectacle lenses, optical adhesive agents and the like. Generally sulfur-containing polymers, particularly polythiourethane, polysulfide and the like are known as such polymers. On the other hand, aromatic polycarbodiimide resins generally have high index of refraction but do not have sufficient properties as the aforementioned optical materials.

The object of the present invention is to provide a polycarbodiimide which has more higher index of refraction than that of the general polycarbodiimide, is excellent in heat stability and has good workability and moldability.

With the aim of achieving the aforementioned object, the present inventors have conducted intensive studies. As a result, it was found that a polycarbodiimide containing 5% by mol or more of naphthalene residue based on the total diisocyanate residues shows far more higher index of refraction than that of the conventionally known polycarbodiimide, thus resulting in the accomplishment of the invention.

Accordingly, the invention is to provide a polycarbodiimide copolymer having a repeating structural unit represented by the following formula (1) in a number "m":

(̵R¹-N=C=N)̵ (1)

(wherein R¹ means a naphthylene group) and a repeating structural unit represented by the following formula (2) in a number "n":

(̵R²-N=C=N)̵ (2)

(wherein R² means an organic diisocyanate residue other than the aforementioned R¹) and also having on both termini a terminal structural unit derived from a monoisocyanate, wherein m + n is from 3 to 200 and n/(m + n) is from 0.05 to 0.99, and a production method thereof. According to the invention, it is desirable that n is an integer of from 3 to 198.

The polycarbodiimide copolymer of the present invention is obtained by carrying out the reaction of naphthalene diisocyanate and other organic diisocyanate with an organic monoisocyanate for controlling the chain length, in an aprotic solvent in the presence of a carbodiimidation catalyst.

The molecular weight of the polycarbodiimide copolymer of the present invention is defined by "m" and "n" described above. The weight average molecular weight of the polycarbodiimide copolymer of the present invention is preferably within the range of from 3,000 to 15,000, more preferably, from 6,000 to 12,000.

### (Organic diisocyanate)

As the organic diisocyanate other than naphthalene diisocyanate, aromatic and aliphatic diisocyanates may be used.

As the aromatic diisocyanate, those which have the following formulae (3) and (4) can be used. 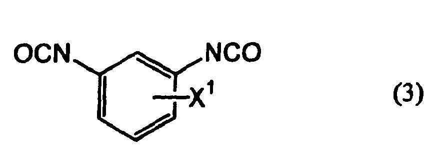 (In the formula, X¹ represents an alkyl group having from 1 to 5 carbon atoms, an alkoxyl group (preferably having from 1 to 2 carbon atoms) or a halogen atom.)

As the diisocyanate having this structure, m-phenylene diisocyanate, 2,4-tolylene diisocyanate, 2,6-tolylene diisocyanate, 6-methoxy-2,4-phenylene diisocyanate, 5-bromo-2,4-tolylene diisocyanate and the like can be exemplified.

Also, another aromatic diisocyanate is 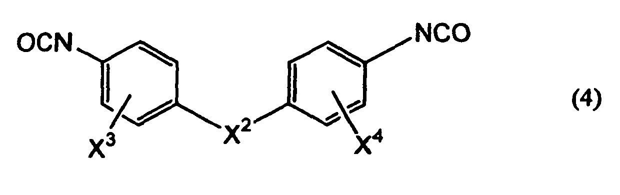 (wherein X² represents a single bond (a direct bond), an alkylene group having from 1 to 5 carbon atoms, oxy group, thio group or sulfinyl group, and each of X³ and X⁴ represents an alkyl group having from 1 to 5 carbon atoms, an alkoxyl group (preferably having from 1 to 2 carbon atoms) or a halogen atom).

As the diisocyanate having this structure, 4,4'-diphenylmethane diisocyanate, 3,3',5,5'-tetraethyl-4,4'-diphenylmethane diisocyanate, 4,4'-diphenylisopropylidene diisocyanate, 4,4'-diphenyl ether diisocyanate, 4,4'-diphenylsulfide diisocyanate, 4,4'-diphenylsulfoxide diisocyanate, 3,3',5,5'-tetramethyl-4,4'-diphenyl diisocyanate, 3,3'-dimethoxy-4,4'-biphenyl diisocyanate, 3,3'-dibromo-4,4'-biphenyl diisocyanate and the like can be exemplified.

In addition, those which have the following formulae (5),(6) and (7) can be used as the aliphatic organic diisocyanate. 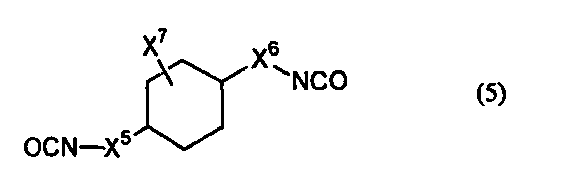 (In this formula, each of X⁵ and X⁶ represents a single bond (a direct bond) or an alkylene group having from 1 to 5 carbon atoms, and X⁷ represents a single bond (a direct bond), an alkyl group having from 1 to 5 carbon atoms or an alkylene group having from 1 to 5 carbon atoms.)

As the diisocyanate having this structure, 4,4'-dicyclohexylmethane diisocyanate, norbornane diisocyanate, 4,4'-cyclohexane diisocyanate, isophorone diisocyanate, methylcyclohexane-2,4-diisocyanate, 2,4-bis(isocyanatomethyl)cyclohexane and the like can be exemplified.

OCN-X⁸-NCO (6)

(In this formula, X⁸ represents an alkylene group having from 1 to 18 carbon atoms.)

As the diisocyanate having this structure, hexamethylene diisocyanate, 2,2,4-trimethylhexamethylene diisocyanate, 2,4,4-trimethylhexamethylene diisocyanate, octamethylene diisocyanate, dodecamethylene diisocyanate and the like can be exemplified. 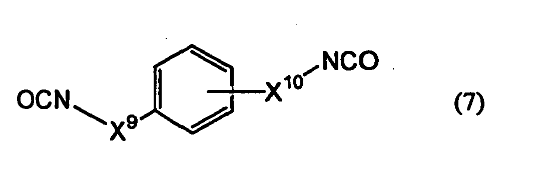 (In this formula, each of X⁹ and X¹⁰ represents a single bond (a direct bond) or an alkylene group having from 1 to 5 carbon atoms.)

As the diisocyanate having this structure, xylylene diisocyanate, α,α,α',α-tetramethylxylylene diisocyanate, 4-isocyanatomethyl-phenyl isocyanate and the like can be exemplified.

### (Monoisocyanate)

As the monoisocyanate for controlling the chain length, for example, phenyl isocyanate, tosyl isocyanate, naphthyl isocyanate, isopropylphenyl isocyanate, methoxyphenyl isocyanate, chlorophenyl isocyanate, an alkyl isocyanate having from 1 to 10 carbon atoms and the like may be cited.

It is desirable to use the monoisocyanate in an amount of from 1 to 10 moles based on 100 moles of the diisocyanate. When the amount of the monoisocyanate to be used is smaller than this range, molecular weight of the obtained polycarbodiimide becomes too large or crosslinking reaction may occur, thus it may cause increase in the solution viscosity or solidification of the solution or generating considerable reduction of storage stability of the polycarbodiimide solution. On the other hand, when amount of the monoisocyanate to be used is larger than the aforementioned range, solution viscosity of the obtained polycarbodiimide may become so low that proper film formation may not be effected in forming a film by coating and drying the solution.

### (Catalyst)

Various kinds of catalyst can be used in the polycarbodiimide polymerization, and their examples include 3-methyl-1-phenyl-2-phosphorene-1-oxide, 1-phenyl-2-phosphorene-1-oxide, 1-phenyl-2-phosphorene-1-sulfide, 1-ethyl-3-methyl-2-phosphorene-1-oxide, 3-methyl-1-phenyl-1-phosphor-3-cyclopentene-1-oxide, 2,5-dihydro-3-methyl-1-phenylphosphol-1-oxide, isomers corresponding thereto and 3-phosphorene. Also can be used are phosphine oxides such as triphenylphosphine oxide, tritolylphosphine oxide, bis(oxadiphenylphosphino)ethane and the like. Regarding the amount of the catalyst, it can be used within the range of from 0.001 to 5% by mol based on the total amount of the isocyanate. Amount of the catalyst when smaller than this may not be practical because of too prolonged period of time for the polymerization. When it exceeds the aforementioned range, on the other hand, the product may be solidified into a gel during the reaction due to too quick reaction, or a product having considerably reduced storage stability might be obtained.

### (Solvent)

According to the polycarbodiimide copolymer of the invention, a polycarbodiimide solution can be obtained by carrying out a carbodiimidation reaction in an aprotic organic solvent. Examples of the aprotic organic solvent to be used for this purpose include benzene, toluene, xylene, trimethylbenzene, tetramethylbenzene, cymene, diethylbenzene and the like alkyl toluene and alkyl benzene; and naphthalene, tetrahydrofuran, dioxane, acetone, butanone, perclene, cyclohexanone, N-methylpyrrolidone, N,N-dimethylformamide, N,N-dimethylacetamide and the like, which may be used alone or as a mixture of two or more, or may contain components which are not concerned in the reaction. Particularly preferred solvents among them are toluene, xylene, benzene, perclene, cyclohexanone, trimethylbenzene, tetramethylbenzene, cymene, diethylbenzene, naphthalene, tetrahydrofuran and dioxane. It is desirable to use these solvents in such an amount that concentration of the polycarbodiimide in the polymer solution becomes from 1 to 90% by weight. When concentration of the polymer solids exceeds this range, the viscosity may become high and storage stability of the solution may also be reduced. On the other hand, in case that the concentration is smaller than the aforementioned range, it is necessary to remove a large amount of the solvent at the time of molding the obtained polymer, which is not practical.

Next, the invention is described further illustratively with reference to examples.

### Example 1

A solution was prepared by mixing and stirring 24.5 g (141 mmol) of tolylene diisocyanate (mixture of isomers: Cosmonate T-80, mfd. by Mitsui Takeda Chemical), 106 g (423 mmol) of 4,4'-diphenylmethane diisocyanate (Cosmonate PH, mfd. by Mitsui Takeda Chemical), 29.6 g (141 mmol) of naphthalene diisocyanate (Cosmonate ND, mfd. by Mitsui Takeda Chemical), 11.9 g (70.6 mmol) of 1-naphthyl isocyanate and 212 g of toluene. This was mixed with 1.36 g (7.0 mmol) of 3-methyl-1-phenyl-2-phosphorene-1-oxide, heated to 80°C while stirring and then kept for 2 hours. Progress of the reaction was verified by an infrared spectroscopic analysis. Illustratively, decrease in the absorption of N-C-O stretching vibration (2270 cm⁻¹) of the isocyanate and increase in the absorption of N-C-N stretching vibration (2135 cm⁻¹) of the carbodiimide were observed. While stirring, the thus obtained polycarbodiimide solution was added dropwise to 1 liter of heptane, and the thus formed precipitate was collected and dried to obtain 132 g of a polymer. A film obtained by casting and drying a toluene solution of this polymer was transparent when observed with the naked eye, and its refractive index was 1.738 at 589 nm when measured by Abbe's refractometer.

The solvent toluene was removed from the thus obtained polycarbodiimide solution at 80°C under a reduced pressure of 10 mmHg. The residual solid matter was hydrolyzed with a potassium hydroxide aqueous solution by the method described in J. Appl. Polym. Sci., 14, 35 (1970) and then extracted with ether. Tolylenediamine, 4,4'-diphenylmethanediamine and 1,5-naphthalenediamine in the resulting ether phase were determined using a gas chromatograph-mass spectrometer (GC-MS). Calibration curves were prepared for the determination using respective standard samples. It was confirmed by this that amounts of tolylenediamine, 4,4'-diphenylmethanediamine and 1,5-naphthalenediamine were in the respective ratio of 20:60:20. It was confirmed by this that the n/(m + n) in the aforementioned formula (1) and formula (2) was 0.80. From this ratio and the weight average molecular weight of 7.8 x 10³ obtained by a GPC of the polycarbodiimide solution, it was able to confirm that m + n is 43.

### Example 2

A solution was prepared by mixing and stirring 15.8 g (91 mmol) of tolylene diisocyanate (mixture of isomers: Cosmonate T-80, mfd. by Mitsui Takeda Chemical), 122 g (491 mmol) of 4,4'-diphenylmethane diisocyanate (Cosmonate PH, mfd. by Mitsui Takeda Chemical), 68.8 g (327 mmol) of naphthalene diisocyanate (Cosmonate ND, mfd. by Mitsui Takeda Chemical), 9.24 g (54.6 mmol) of 1-naphthyl isocyanate and 170 g of toluene. This was mixed with 0.87 g (4.5 mmol) of 3-methyl-1-phenyl-2-phosphorene-1-oxide, heated to 80°C while stirring and then kept for 2 hours. Progress of the reaction was verified by an infrared spectroscopic analysis. Illustratively, decrease in the absorption of N-C-O stretching vibration (2270 cm⁻¹) of the isocyanate and increase in the absorption of N-C-N stretching vibration (2135 cm⁻¹) of the carbodiimide were observed. While stirring, the thus obtained polycarbodiimide solution was added dropwise to 1 liter of heptane, and the thus formed precipitate was collected and dried to obtain 138 g of a polymer. A film obtained by casting and drying a toluene solution of this polymer was transparent when observed with the naked eye, and its refractive index was 1.744 at 589 nm when measured by Abbe's refractometer.

The thus obtained polycarbodiimide solution was treated in the same manner as in Example 1 to carry out each determination. As a result, it was confirmed that amounts of tolylenediamine, 4,4'-diphenylmethanediamine and 1,5-naphthalenediamine were in the respective ratio of 10:54:36. It was confirmed by this that the n/(m + n) in the aforementioned formula (1) and formula (2) was 0.64. From this ratio and the weight average molecular weight of 8.2 x 10³ obtained by a GPC of the polycarbodiimide solution, it was able to confirm that m + n is 46.

### Example 3

A solution was prepared by mixing and stirring 29.8 g (171 mmol) of tolylene diisocyanate (mixture of isomers: Cosmonate T-80, mfd. by Mitsui Takeda Chemical), 94.4 g (377 mmol) of 4,4'-diphenylmethane diisocyanate (Cosmonate PH, mfd. by Mitsui Takeda Chemical), 64.9 g (308 mmol) of naphthalene diisocyanate (Cosmonate ND, mfd. by Mitsui Takeda Chemical), 8.71 g (51.4 mmol) of 1-naphthyl isocyanate and 184 g of toluene. This was mixed with 0.82 g (4.2 mmol) of 3-methyl-1-phenyl-2-phosphorene-1-oxide, heated to 80°C while stirring and then kept for 2 hours. Progress of the reaction was verified by an infrared spectroscopic analysis. Illustratively, decrease in the absorption of N-C-O stretching vibration (2270 cm⁻¹) of the isocyanate and increase in the absorption of N-C-N stretching vibration (2135 cm⁻¹) of the carbodiimide were observed. While stirring, the thus obtained polycarbodiimide solution was added dropwise to 1 liter of heptane, and the thus formed precipitate was collected and dried to obtain 140 g of a polymer. A film obtained by casting and drying a toluene solution of this polymer was transparent when observed with the naked eye, and its refractive index was 1.757 at 589 nm when measured by Abbe's refractometer.

The thus obtained polycarbodiimide solution was treated in the same manner as in Example 1 to carry out each determination. As a result, it was confirmed that amounts of tolylenediamine, 4,4'-diphenylmethanediamine and 1,5-naphthalenediamine were in the respective ratio of 20:44:36. It was confirmed by this that the n/(m + n) in the aforementioned formula (1) and formula (2) was 0.64. From this ratio and the weight average molecular weight of 7.9 x 10³ obtained by a GPC of the polycarbodiimide solution, it was able to confirm that m + n is 45.

The polycarbodiimide of the invention has a refractive index more higher than that of the general polycarbodiimide, and is excellent in heat stability, workability and moldability. In addition, since this polymer is obtained in a half-hardened film form, it can be used in new fields such as a lens sheet by press working.

## Claims (Claims for the following Contracting State(s): AT, BE, BG, CH, CY, CZ, DK, EE, ES, FI, FR, GR, HU, IE, IT, LI, LU, MC, NL, PT, RO, SE, SI, SK, TR)

1. A polycarbodiimide copolymer having a repeating structural unit represented by the following formula (1) in a number "m":
(̵R¹-N=C=N)̵ (1)
(wherein R¹ means a naphthylene group) and a repeating structural unit represented by the following formula (2) in a number "n":
(̵R²-N=C=N)̵ (2)
(wherein R² means an organic diisocyanate residue other than the aforementioned R¹) and also having on both termini a terminal structural unit derived from a monoisocyanate, wherein m + n is from 3 to 200 and n/(m + n) is from 0.05 to 0.99.

2. The polycarbodiimide copolymer according to claim 1, wherein n in the aforementioned formula is an integer of from 3 to 198.

3. A solution of a polycarbodiimide copolymer, comprising an aprotic organic solvent and the polycarbodiimide copolymer of claim 1 dissolved therein.

4. A solution of a polycarbodiimide copolymer, comprising an aprotic organic solvent and the polycarbodiimide copolymer of claim 2 dissolved therein.

5. A method for producing a polycarbodiimide copolymer, which comprises carrying out carbodiimidation reaction of an organic diisocyanate and a monoisocyanate in the presence of a carbodiimidation catalyst, wherein the reaction is carried out at a temperature of from 0 to 120°C using 5% by mol or more of naphthalene diisocyanate based on the total organic isocyanate.

## Claims (Claims for the following Contracting State(s): DE, GB)

1. A polycarbodiimide copolymer having a repeating structural unit represented by the following formula (1) in a number "m":
(̵R¹-N=C=N)̵ (1)
wherein R¹ means a naphthylene group; and
a repeating structural unit represented by the following formula (2) in a number "n":
(̵R¹-N=C=N)̵ (2)
wherein R² means an organic diisocyanate residue other than the aforementioned R¹ and also having on both termini a terminal structural unit derived from a monoisocyanate, wherein m + n is from 3 to 200 and n/(m + n) is from 0.05 to 0.99,
with the proviso that
i) the product from the reaction of 29.89 g (171.6 mmol) of tolylene diisocyanate, 94.89 g (377.52 mmol) of 4,4'-diphenylmethane diisocyanate, 64.92 g (308.88 mmol) of naphthalene diisocyanate and 8.71 g (51.48 mmol) of 1-naphthyl isocyanate; and
ii) the product from the reaction of 89.01 g (355.68 mmol) of 4,4'-diphenylmethane diisocyanate, 24.92 g (118.56 mmol) of naphthalene diisocyanate, 44.87 g (266.76 mmol) of hexamethylene diisocyanate and 7.52 g (44.46 mmol) of 1-naphthyl isocyanate
are excluded.

2. The polycarbodiimide copolymer according to claim 1, wherein n in the aforementioned formula is an integer of from 3 to 198.

3. A solution of a polycarbodiimide copolymer, comprising an aprotic organic solvent and the polycarbodiimide copolymer of claim 1 dissolved therein.

4. A solution of a polycarbodiimide copolymer, comprising an aprotic organic solvent and the polycarbodiimide copolymer of claim 2 dissolved therein.

5. A method for producing the polycarbodiimide copolymer of claim 1, which comprises carrying out carbodiimidation reaction of an organic diisocyanate and a monoisocyanate in the presence of a carbodiimidation catalyst, wherein the reaction is carried out at a temperature of from 0 to 120°C using 5% by mol or more of naphthalene diisocyanate based on the total organic isocyanate.

## Patentansprüche (Patentansprüche für folgende(n) Vertragsstaat(en): AT, BE, BG, CH, CY, CZ, DK, EE, ES, FI, FR, GR, HU, IE, IT, LI, LU, MC, NL, PT, RO, SE, SI, SK, TR)

1. Polycarbodiimidcopolymer, das eine sich wiederholende Struktureinheit, die durch die folgende Formel (1) wiedergegeben ist, in einer Anzahl "m" aufweist:
(̵R¹-N=C=N)̵ (1)
wobei R¹ eine Naphthylengruppe bedeutet, und eine sich wiederholende Struktureinheit, die durch die folgende Formel (2) wiedergegeben ist, in einer Anzahl "n" aufweist:
(̵R²-N=C=N)̵ (2)
wobei R² einen organischen Diisocyanatrest bedeutet, der von dem vorstehend erwähnten R¹ verschieden ist, und auch an beiden Enden eine endständige Struktureinheit aufweist, die von einem Monoisocyanat abgeleitet ist, wobei m + n 3 bis 200 beträgt und n / (m + n) 0,05 bis 0,99 beträgt.

2. Polycarbodiimidcopolymer nach Anspruch 1, wobei n in der vorstehend erwähnten Formel eine ganze Zahl von 3 bis 198 ist.

3. Lösung von einem Polycarbodiimidcopolymer, umfassend ein aprotisches organisches Lösungsmittel und das darin gelöste Polycarbodiimidcopolymer nach Anspruch 1.

4. Lösung von einem Polycarbodiimidcopolymer, umfassend ein aprotisches organisches Lösungsmittel und das darin gelöste Polycarbodiimidcopolymer nach Anspruch 2.

5. Verfahren zum Herstellen eines Polycarbodiimidcopolymers, welches das Ausführen einer Carbodiimidisierungsreaktion eines organischen Diisocyanats und eines Monoisocyanats in Gegenwart eines Carbodiimidisierungskatalysators umfasst, wobei die Reaktion bei einer Temperatur von 0 bis 120 °C unter Verwendung von 5 mol% oder mehr Naphthalindiisocyanat, bezogen auf das gesamte organische Isocyanat, ausgeführt wird.

## Patentansprüche (Patentansprüche für folgende(n) Vertragsstaat(en): DE, GB)

1. Polycarbodiimidcopolymer, das eine sich wiederholende Struktureinheit, die durch die folgende Formel (1) wiedergegeben ist, in einer Anzahl "m" aufweist:
(̵R¹-N=C=N)̵ (1)
wobei R¹ eine Naphthylengruppe bedeutet, und
eine sich wiederholende Struktureinheit, die durch die folgende Formel (2) wiedergegeben ist, in einer Anzahl "n" aufweist:
(̵R²-N=C=N)̵ (2)
wobei R² einen organischen Diisocyanatrest bedeutet, der von dem vorstehend erwähnten R¹ verschieden ist, und auch an beiden Enden eine endständige Struktureinheit aufweist, die von einem Monoisocyanat abgeleitet ist, wobei m + n 3 bis 200 beträgt und n / (m + n) 0,05 bis 0,99 beträgt,
mit der Maßgabe, dass
i) das Produkt aus der Reaktion von 29,89 g (171,6 mmol) Tolylendiisocyanat, 94,89 g (377,52 mmol) 4,4'-Diphenylmethandiisocyanat, 64,92 g (308,88 mmol) Naphthalindiisocyanat und 8,71 g (51,48 mmol) 1-Naphthylisocyanat; und
ii) das Produkt aus der Reaktion von 89,01 g (355,68 mmol) 4,4'-Diphenylmethandiisocyanat, 24,92 g (118,56 mmol) Naphthalindiisocyanat, 44,87 g (266,76 mmol) Hexamethylendiisocyanat und 7,52 g (44,46 mmol) 1-Naphthylisocyanat
ausgeschlossen sind.

2. Polycarbodiimidcopolymer nach Anspruch 1, wobei n in der vorstehend erwähnten Formel eine ganze Zahl von 3 bis 198 ist.

3. Lösung von einem Polycarbodiimidcopolymer, umfassend ein aprotisches organisches Lösungsmittel und das darin gelöste Polycarbodiimidcopolymer nach Anspruch 1.

4. Lösung von einem Polycarbodiimidcopolymer, umfassend ein aprotisches organisches Lösungsmittel und das darin gelöste Polycarbodiimidcopolymer nach Anspruch 2.

5. Verfahren zum Herstellen des Polycarbodiimidcopolymers nach Anspruch 1, welches das Ausführen einer Carbodiimidisierungsreaktion eines organischen Diisocyanats und eines Monoisocyanats in Gegenwart eines Carbodiimidisierungskatalysators umfasst, wobei die Reaktion bei einer Temperatur von 0 bis 120 °C unter Verwendung von 5 mol% oder mehr Naphthalindiisocyanat, bezogen auf das gesamte organische Isocyanat, ausgeführt wird.

## Revendications (Revendications pour l'(les) Etat(s) contractant(s) suivant(s): AT, BE, BG, CH, CY, CZ, DK, EE, ES, FI, FR, GR, HU, IE, IT, LI, LU, MC, NL, PT, RO, SE, SI, SK, TR)

1. Copolymère de polycarbodiimide présentant une unité de structure répétitive représentée par la formule (1) suivante dans un nombre "m" :
(̵R¹-N=C=N)̵ (1)
(dans laquelle R¹ indique un groupe naphtylène) et
une unité de structure répétitive représentée par la formule suivante (2) dans un nombre "n" :
(̵R²-N=C=N)̵ (2)
(dans laquelle R² indique un résidu diisocyanate organique différent du R¹ cité précédemment) et présentant également sur les deux terminaisons une unité de structure terminale dérivée d'un monoisocyanate, où m + n est égal à de 3 à 200 et n/(m + n) est égal à de 0,05 à 0,99.

2. Copolymère de polycarbodiimide selon la revendication 1, dans lequel n dans la formule citée précédemment est un nombre entier de 3 à 198.

3. Solution d'un copolymère de polycarbodiimide comprenant un solvant organique aprotique et le copolymère de polycarbodiimide selon la revendication 1 dissous dans celui-ci.

4. Solution d'un copolymère de polycarbodiimide comprenant un solvant organique aprotique et le copolymère de polycarbodiimide selon la revendication 2 dissous dans celui-ci.

5. Procédé de production d'un copolymère de polycarbodiimide qui comprend la réalisation d'une réaction de carbodiimidation d'un diisocyanate organique et d'un monoisocyanate en présence d'un catalyseur de carbodiimidation, dans lequel la réaction est réalisée à une température de 0 à 120°C en utilisant 5 % en mole ou plus de diisocyanate de naphtalène rapporté à l'isocyanate organique total.

## Revendications (Revendications pour l'(les) Etat(s) contractant(s) suivant(s): DE, GB)

1. Copolymère de polycarbodiimide présentant une unité de structure répétitive représentée par la formule (1) suivante dans un nombre "m":
(̵R¹-N=C=N)̵ (1)
dans laquelle R¹ indique un groupe naphtylène ; et
une unité de structure répétitive représentée par la formule suivante (2) dans un nombre "n" :
(̵R²-N=C=N)̵ (2)
dans laquelle R² indique un résidu dilsocyanate organique différent du R¹ cité précédemment et présentant également sur les deux terminaisons une unité de structure terminale dérivée d'un monoisocyanate, où m + n est égal à de 3 à 200 et n/(m + n) est égal à de 0,05 à 0,99,
à condition que
i) le produit de la réaction de 29,89 g (171,6 mmol) de diisocyanate de tolylène, de 94,89 g (377,52 mmol) de diisocyanate de 4,4'-diphénylméthane, de 64,92 g (308,88 mmol) de diisocyanate, de naphtalène et de 8,71 g (51,48 mmol) d'isocyanate de 1-naphtyle ; et
ii) le produit de la réaction de 89,01 g (355,68 mmol) de diisocyanate de 4,4'-diphénylméthane, de 24,92 g (118,56 mmol) de diisocyanate de naphtalène, de 44,87 g (266,76 mmol) de diisocyanate d'hexaméthylène et de 7,52 g (44,46 mmol) d'isocyanate de 1-naphtyle
soient exclus.

2. Copolymère de polycarbodiimide selon la revendication 1, dans lequel n dans la formule citée précédemment est un nombre entier de 3 à 198.

3. Solution d'un copolymère de polycarbodiimide comprenant un solvant organique aprotique et le copolymère de polycarbodiimide selon la revendication 1 dissous dans celui-ci.

4. Solution d'un copolymère de polycarbodiimide comprenant un solvant organique aprotique et le copolymère de polycarbodiimide selon la revendication 2 dissous dans celul-ci.

5. Procédé de production du copolymère de polycarbodiimide selon la revendication 1 qui comprend la réalisation d'une réaction de carbodiimidation d'un diisocyanate, organique et d'un monoisocyanate en présence d'un catalyseur de carbodiimidation, dans lequel la réaction est réalisée à une température de 0 à 120°C en utilisant 5 % en mole ou plus de diisocyanate de naphtalène rapporté à l'isocyanate organique total.
